# FASCICULE DE BREVET EUROPEEN

(11) **EP 3 877 370 B1**
(45) Date de publication et mention de la délivrance du brevet: **02.08.2023**
(21) Numéro de dépôt: 19818226.3
(22) Date de dépôt: 07.11.2019
(51) Int. Cl.: C07D 257/02, C25B 11/04

(54) **DÉRIVÉS DE CYCLAM IMMOBILISÉS SUR SUPPORT SOLIDE ET LEURS UTILISATIONS POUR LA CATALYSE ET/OU POUR LA DÉTECTION D'IONS**
AUF EINEM FESTEN TRÄGER IMMOBILISIERTE CYCLAMENDERIVATE UND IHRE VERWENDUNGEN ZUR KATALYSE UND/ODER ZUM NACHWEIS VON IONEN
CYCLAM DERIVATIVES IMMOBILISED ON A SOLID SUPPORT AND USES THEREOF FOR CATALYSIS AND/OR FOR THE DETECTION OF IONS

(30) Priorité: 09.11.2018 FR 1860399
(43) Date de publication de la demande: 15.09.2021
(73) Titulaire: Université de Bretagne Occidentale, 29200 Brest (FR); Centre National de la Recherche Scientifique - CNRS, 75016 Paris (FR)
(72) Inventeur: LE POUL, Nicolas, 29810 Ploumoguer (FR); FORGET, Amélie, 29200 Brest (FR); TRIPIER, Raphaël, 29860 Kersaint Plabennec (FR); BERNARD, Hélène, 29880 Plouguerneau (FR)
(74) Mandataire: Plasseraud IP
(86) Numéro de dépôt international: PCT/FR2019/052658
(87) Numéro de publication internationale: WO 2020/094999

(56) Documents cités:
- JASMIN JEAN-PHILIPPE ET AL: "Straightforward grafting approach for cyclam-functionalized screen-printed electrodes for selective Cu(II) determination", ELECTROCHIMICA ACTA, vol. 200, 2016, pages 115-122, XP029519883, ISSN: 0013-4686, DOI: 10.1016/J.ELECTACTA.2016.03.141
- GAIA NERI ET AL: "A functionalised nickel cyclam catalyst for CO2 reduction: electrocatalysis, semiconductor surface immobilisation and light-driven electron transfer", PHYSICAL CHEMISTRY CHEMICAL PHYSICS, vol. 17, no. 3, 2015, pages 1562-1566, XP055580044, ISSN: 1463-9076, DOI: 10.1039/C4CP04871G

## Description

La présente invention a pour objet des dérivés de cyclam immobilisés sur support solide et leurs utilisations pour la catalyse et/ou pour la détection d'ions.

Le cyclam désigne plus particulièrement une polyamine cyclique dénommée « 1,4,8,11-tétraazacyclotétradécane », de formule (NHCH₂CH₂NHCH₂CH₂CH₂)₂. Grâce aux doublets libres de ses amines secondaires, ces macrocycles azotés peuvent former des complexes très stables avec de nombreux cations et notamment les métaux de transition.

La capacité chélatante de ces composés peut être améliorée ou encore être étendue à d'autres métaux tels que les lanthanides ou les métaux lourds, à l'aide de groupements fonctionnels et en particulier de ligands particuliers, présentant une affinité avec ces cations ou métaux. En fonctionalisant ces macrocycles, c'est-à-dire en y greffant un ou plusieurs groupements fonctionnels, notamment coordinants (ligands), il est possible de modifier les propriétés complexantes du macrocycle, sa solubilité, son affinité et sa sélectivité par rapport à un élément donné.

Les dérivés de cyclam ainsi obtenus (c'est-à-dire fonctionnalisés par des groupements fonctionnels) peuvent être utilisés dans de nombreuses applications, telles que la catalyse, l'épuration de liquides et de gaz, les méthodes d'analyse et de détection etc.

Il a déjà été proposé dans la littérature d'immobiliser (c'est-à-dire de greffer) des dérivés de cyclam sur des supports solides, notamment pour des applications en catalyse ou en détection d'ions.

Ainsi les auteurs C. P. Kubiak et al. ⁽¹⁾ décrivent, pour leurs applications en catalyse, des dérivés de Ni(cyclam) fonctionnalisés au niveau d'un des azotes par des groupements alkynyl [Ni(alkynyl-cyclam)]²⁺, lesdits dérivés étant greffés sur des électrodes de carbone vitreux.

A. Chaussé et al. ⁽²⁾ décrivent, pour leurs applications en détection d'ions Cu²⁺, des dérivés de cyclam fonctionnalisés au niveau d'un des azotes par un p-aminobenzyl, lesdits dérivés étant greffés, par voie diazonium, sur des électrodes de carbone sérigraphiées.

J. Cowan et al. ⁽³⁾ décrivent, pour leurs applications en catalyse, des dérivés de Ni(cyclam) fonctionnalisés au niveau d'un des carbones par un groupement acide carboxylique [Ni(cyclam-COOH)]²⁺, lesdits dérivés étant greffés sur des nanoparticules de TiO₂.

L'un des buts de l'invention est de trouver de nouvelles structures de dérivés de cyclam greffés sur des supports solides, qui soient particulièrement avantageuses pour des applications en catalyse et/ou pour la détection d'ions.

A titre d'exemple d'application en catalyse des structures de l'invention, on pourra citer la réduction électrocatalytique du dioxyde de carbone (CO₂) en milieu aqueux.

L'augmentation continue de la teneur en CO₂ en milieux atmosphérique et marin est devenue l'une des préoccupations principales pour la société ces dernières années, en raison des conséquences environnementales et sociétales liées à l'effet de serre. Pour diminuer cette augmentation, une stratégie actuelle consiste à développer des dispositifs catalytiques peu onéreux et respectueux de l'environnement pouvant réduire de manière efficace le CO₂ en matières premières (CO, HCO₂H, CH₃OH) utilisables par l'industrie. De nombreux catalyseurs moléculaires homogènes à base de métaux de transition ont été développés depuis plus de trente ans ^{(4,5,6)}.

Cependant, l'activation du CO₂ par des catalyseurs hétérogènes moléculaires reste peu développée. Une des raisons principales est la difficulté de concevoir des catalyseurs à faible surtension d'activation, qui puissent être utilisables en milieu aqueux. En outre, le catalyseur hétérogène doit être sélectif non seulement vis-à-vis de la réaction de dégagement d'hydrogène, mais aussi vis-à-vis des sous-produits dérivés du CO₂.

Les travaux des Inventeurs se sont plus particulièrement centrés sur des dérivés de cyclam fonctionnalisés au niveau d'un atome de carbone (C-fonctionnalisation) du macrocycle. En effet, un macrocycle azoté fonctionnalisé au niveau d'un atome de carbone est un macrocyle bifonctionnel, capable 1/ d'une part de chélater un cation (fonction de coordination) et de conserver les propriétés de chelation identiques au composé non bifonctionnel, ce qui n'est pas le cas d'une N-fonctionnalisation, et 2/ d'autre part d'être greffé sur un élément donné tel qu'un support solide (fonction de couplage) sans modifier les propriétés chélatantes du macrocyle ou dénaturer la structure et les propiétés d'autres ligands reliés au macrocycle.

Un autre but de l'invention est de trouver de nouvelles structures de dérivés de cyclam dans lesquelles les dérivés de cyclam sont immobilisés (greffés) de façon covalente sur des supports solides, et plus particulièrement dans lesquelles les dérivés de cyclam forment une liaison carbone-carbone ou carbone-hétéroatome stable avec le support solide.

Outre la grande stabilité d'une telle structure, le greffage covalent confère également à ladite structure la capacité d'être utilisée dans différents types de milieux : aqueux/organiques, oxydants/réducteurs etc.

Un autre but de l'invention est de trouver une procédure d'immobilisation des dérivés de cyclam sur le support qui soit simple à mettre en oeuvre et qui ne requiert que des conditions douces en termes de solvant (aqueux), pH (faiblement acide ou basique), température (proche de la température ambiante), pression (1 atm.) et potentiel appliqué à l'électrode (-1.0 V < *E*ₐₚₚ < 1.0 V vs. Ag/AgCl).

Après d'intenses recherches menées par les Inventeurs ces derniers ont mis au point de nouvelles structures de dérivés de cyclam C-fonctionnalisés immobilisés sur des supports solides et répondant aux buts recherchés par les Inventeurs.

Selon un premier objet, l'invention concerne de nouvelles structures comprenant des dérivés de cyclam C-fonctionnalisés immobilisés de façon covalente sur un support solide.

Selon un deuxième objet, l'invention concerne l'utilisation de ces structures en catalyse, notamment pour la réduction électrocatalytique du dioxyde de carbone (CO₂) en milieu aqueux, et/ou pour la détection d'ions.

La présente invention a plus particulièrement pour objet un produit caractérisé en ce qu'il présente la structure de formule générale (I) suivante : 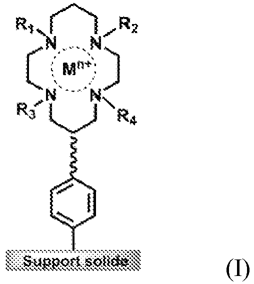 dans laquelle
- R₁, R₂, R₃ et R₄ représentent indépendamment l'un de l'autre H ; un alkyle en C₁-C₅ ; (CH₂)ₘR' avec m égal à un entier allant de 0 à 3 et R' représentant C₆H₅ (Ph), NO₂, CO₂(alkyle en C₁-C₃), O(alkyle en C₁-C₃), un halogène choisi parmi Cl, F, Br ou I ;
- « Support solide » représente un matériau conducteur, notamment une électrode ou des nanoparticules ;
- la liaison « 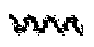 » entre le carbone du macrocycle 1,4,8,11-tétraazacyclotétradécane (cyclam) et le carbone du groupement C₆H₄ représente le radical divalent -(CH₂)ₘ- avec m égal à un entier allant de 0 à 3, et est de préférence égal à 1 ; -CO-NH- ; -NH-CO- ;
- la liaison « » entre le carbone du groupement C₆H₄ et le support solide est une liaison covalente ;
- Mⁿ⁺, s'il est présent, représente un cation métallique, avec n qui est un entier allant de 1 à 4 et M est choisi dans le groupe comprenant Ni, Cu, Hg, Zn, Co, Cd et Pb.

Selon un mode de réalisation avantageux de l'invention, dans la structure de formule (I) telle que ci-dessus définie, R₁, R₂, R₃ et R₄ représentent indépendamment l'un de l'autre H ou un alkyle en C₁-C₅, ledit alkyle étant de préférence un méthyle.

A titre d'exemple, dans la structure de formule (I), R₁, R₂, R₃ et R₄ représentent chacun un atome d'hydrogène.

Toujours à titre d'exemple, dans la structure de formule (I), R₁, R₂, R₃ et R₄ représentent chacun un méthyle.

Selon un autre mode de réalisation avantageux, dans la structure de formule (I) telle que ci-dessus définie, le « support solide » est une électrode choisie dans le groupe comprenant une électrode de carbone, d'or, de platine, d'oxyde d'étain dopé au fluor (« FTO » acronyme anglais de « Fluorine doped Tin oxide »), d'oxyde d'étain dopé à l'indium (« ITO » acronyme anglais de « Indium doped Tin oxide »), et est de préférence une électrode de carbone ou d'or.

A titre d'exemples d'électrodes de carbone, on pourra citer le carbone vitreux (« GC » acronyme anglais de « Glassy Carbon »), des films de photorésist pyrolysé (« PPF » acronyme anglais de « Photopyrolyzed Photoresist Films »), le diamant dopé au bore (« BDD » acronyme anglais de « Bore-Doped Diamond »), le graphène, etc.

Selon un autre mode de réalisation avantageux, le support solide est une nanoparticule, par exemple une nanoparticule d'or ou de carbone.

Selon un autre mode de réalisation avantageux de l'invention, dans la structure de formule (I) telle que ci-dessus définie, la liaison covalente « » entre le carbone du groupement C₆H₄ et le support solide est une liaison de type « carbone-carbone » ou « carbone-hétéroatome », ledit hétéroatome étant un métal, de préférence l'or.

Selon l'invention, dans la structure de formule (I) telle que ci-dessus définie, 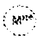 est représenté en pointillés ce qui signifie qu'il peut être présent ou absent de la structure.

Lorsque Mⁿ⁺ est présent, la structure de formule générale (I) de l'invention est alors plus particulièrement représentée par la structure de formule générale (II) suivante : 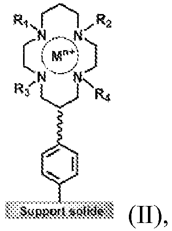 dans laquelle R₁, R₂, R₃, R₄, le « support solide », Mⁿ⁺ et la liaison « 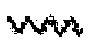 » sont tels que ci-dessus définis.

La structure de formule (II) répond donc à la structure de formule (I) dans laquelle Mⁿ⁺ est présent.

Lorsque Mⁿ⁺ est absent, la structure de formule générale (I) de l'invention est alors plus particulièrement représentée par la structure de formule générale (III) suivante : 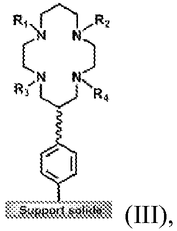 dans laquelle R₁, R₂, R₃, R₄, le « support solide » et la liaison « 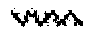 » sont tels que ci-dessus définis.

La structure de formule (III) répond donc à la structure de formule (I) dans laquelle Mⁿ⁺ est absent.

Selon un mode de réalisation particulier, Mⁿ⁺ est présent et représente un cation métallique choisi dans le groupe comprenant Ni²⁺ , Cu²⁺ , Hg²⁺, Zn²⁺, Co²⁺ _{>} Cd²⁺ et Pb²⁺, et est de préférence Ni²⁺ ou Cu²⁺.

Selon encore un mode de réalisation particulier de l'invention, la liaison « 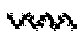 » entre le carbone du cyclam et le carbone du groupement C₆H₄ est le radical divalent -CH₂-.

A titre d'exemples plus particuliers de produits de l'invention, on pourra citer ceux répondant à la structure de formule (I) dans laquelle :
- R₁, R₂, R₃ et R₄ représentent indépendamment l'un de l'autre H ou un alkyle en C₁-C₅, ledit alkyle étant de préférence un méthyle ;
- le « support solide » est une électrode de carbone ou d'or ;
- Mⁿ⁺, s'il est présent, représente un cation métallique, et est de préférence Ni²⁺ ou Cu²⁺ ;
- la liaison « 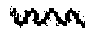 » est le radical divalent -CH₂-.

Selon un mode de réalisation particulier de l'invention, dans la structure de formule (I) telle que ci-dessus définie, Mⁿ⁺ est présent, et est alors plus particulièrement représenté par la structure de formule (II) telle que ci-dessus définie.

Un tel produit de formule (II) pourra avantageusement être utilisé pour une application en catalyse.

La présente invention a donc également pour objet l'utilisation d'un produit de formule (II) tel que défini ci-dessus, pour la catalyse et plus particulièrement l'électrocatalyse.

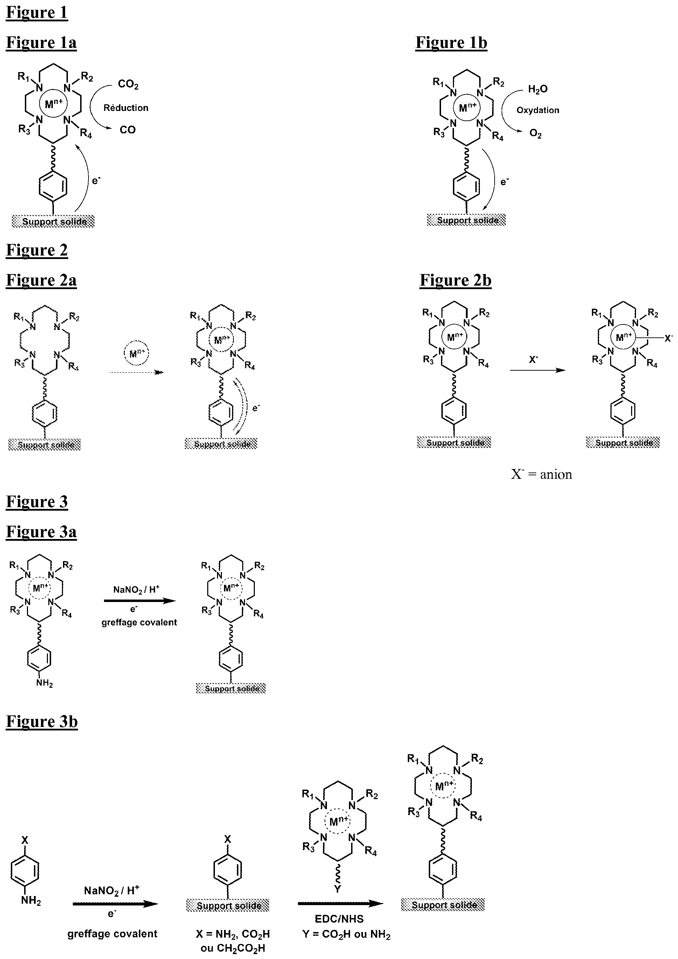

Une telle utilisation est schématisée à la **figure 1****.**

Les propriétés d'oxydoréduction de cations métalliques Mⁿ⁺ tels que Ni²⁺ ou Cu²⁺ etc. insérés dans le cyclam sont en effet exploitables pour des applications en électrocatalyse de molécules « cibles » telles que le dioxyde de carbone (CO₂), l'eau (H₂O), le dioxygène (O₂) etc.

Le principe repose sur une interaction favorable de ces molécules « cibles » avec les complexes greffés sur support solide, à savoir les produits de formule (II) de l'invention, sous contrôle du potentiel électrochimique appliqué (catalyseur hétérogène).

Selon un mode de réalisation avantageux, le produit de formule (II) de l'invention est un catalyseur utilisé pour la réduction électrocatalytique en milieu aqueux du dioxyde de carbone (CO₂) en monoxyde de carbone (CO).

Une telle utilisation est schématisée à la **figure 1a****.** Mⁿ⁺ est tel que ci-dessus défini.

A titre d'exemple de Mⁿ⁺ on pourra citer Ni²⁺ car un des avantages du « Ni-cyclam » est sa sélectivité pour la réduction du CO₂ conduisant quasi-exclusivement à un seul produit de réaction, à savoir le CO. Cette propriété est un atout potentiel pour la valorisation du CO₂ par les industries.

Selon un autre mode de réalisation avantageux de l'invention, le produit de formule (II) est un catalyseur utilisé pour l'oxydation électrocatalytique de l'eau (H₂O) en dioxygène (O₂).

Une telle utilisation est schématisée à la **figure 1b****.** Mⁿ⁺ est tel que ci-dessus défini. A titre d'exemple de Mⁿ⁺ on pourra citer Cu²⁺.

Concernant le produit de formule (II), outre son application comme catalyseur, on pourra également citer son application comme détecteur et/ou capteur d'anions «X⁻ », par coordination du cation Mⁿ⁺ inséré dans le cyclam à l'anion X⁻ ou vice versa (par coordination de l'anion X⁻ au cation Mⁿ⁺).

Une telle utilisation est schématisée à la **figure 2b****.**

L'invention a donc encore pour objet l'utilisation d'un produit de formule (II) pour la détection et/ou capture d'anions choisis dans le groupe comprenant des halogénures, à savoir Cl⁻, Br⁻ et F⁻ ; des ions nitrate (NO₃⁻) ; des ions hydrogénophosphate (HPO₄²⁻) et des ions sulfate (SO₄²⁻).

Les anions ci-dessus mentionnés sont plus particulièrement des anions non électroactifs en solution aqueuse.

Une telle application de détection et/ou capture peut être utilisée pour différents milieux (gaz/liquide) selon l'application visée, telle que par exemple la détection d'anions polluants à l'état de traces.

Selon encore un autre mode de réalisation particulier de l'invention, dans la structure de formule (I) telle que ci-dessus définie Mⁿ⁺ est absent et est alors plus particulièrement représenté par la structure de formule (III) telle que ci-dessus définie.

Un tel produit de formule (III) pourra avantageusement être utilisé pour une application pour la détection et/ou capture de cations Mⁿ⁺.

Une telle utilisation est schématisée à la **figure 2a****.**

La forte affinité des cyclam pour certains cations métalliques Mⁿ⁺ peut en effet être exploitée pour la détection spécifique de ces cations par voie électrochimique et/ou spectroscopique. La C-fonctionnalisation du cyclam permet de moduler la nature des substituants R₁, R₂, R₃ et R₄ sur le cycle azoté du cyclam et ainsi de cibler le cation métallique à détecter.

Cette propriété est également utilisée pour la détection d'anions telle que décrite ci-dessus, par coordination dudit anion au cation métallique Mⁿ⁺ inséré dans le cyclam ou vice-versa.

L'invention a encore pour objet l'utilisation d'un produit de formule (III) tel que défini ci-dessus pour la détection et/ou capture de cations Mⁿ⁺ dans lesquels n est un entier allant de 1 à 4 et M est choisi dans le groupe comprenant Ni, Cu, Hg, Zn, Co, Cd et Pb, et de préférence Ni²⁺ ou Cu²⁺.

Le procédé de préparation des produits de formule (I) de l'invention consiste à utiliser et à adapter une méthode de greffage électrochimique « par voie diazonium » qui est basée sur la formation *in-situ* de dérivés diazonium (voir **figure 3**).

Les Inventeurs ont ainsi réussi à adapter, dans des conditions optimales, cette méthode électrochimique par voie diazonium, à des dérivés cyclam C-fonctionnalisés, pour aboutir à la formation de surfaces (couches) très stables (grâce à une liaison covalente) par réaction du radical aryle avec la surface de l'électrode ^{(7,8,9)}.

Dans cette optique, deux stratégies de greffage ont été utilisées par les Inventeurs, à savoir :
1/ le greffage direct sur support solide de dérivés cyclam-diazonium formés *in-situ* à partir de dérivés cyclam C-aniline (**figure 3a**) ;
2/ le greffage en deux étapes sur support solide de dérivés cylam ; une première étape de greffage sur support solide par voie diazonium du 1,4-diaminobenzène (qui possède une fonction terminale NH₂), d'acide p-amino benzoïque ou d'acide p-amino phényléthanoïque (qui possèdent chacun une fonction terminale acide carboxylique COOH) puis une seconde étape d'accroche avec un dérivé cyclam C-acide carboxylique (qui possède une fonction COOH) ou un dérivé cyclam C-amine (qui possède une fonction NH₂) par couplage peptidique selon un protocole EDC/NHS classique (EDC = 1-éthyl-3-(3-diméthylaminopropyl)carbodiimide et NHS = N-hydroxysuccinimide) (**figure 3b**).

La **figure 1** représente l'utilisation d'un produit de l'invention de formule générale (II) comme catalyseur. Plus particulièrement, la **figure 1a** représente son utilisation pour la réduction électrocatalytique du dioxyde de carbone (CO₂) en monoxyde de carbone (CO). La **figure 1b** représente son utilisation pour l'oxydation électrocatalytique de l'eau (H₂O) en dioxygène (O₂).

La **figure 2** représente l'utilisation d'un produit de l'invention de formule générale (I) comme détecteur et/ou capteur d'ions. Plus particulièrement, la **figure 2a** représente la détection et/ou capture d'un cation Mⁿ⁺ à l'aide d'un produit de formule générale (III). La **figure 2b** représente la détection et/ou capture d'un anion X⁻, à l'aide d'un produit de formule générale (II), par coordination de l'anion X⁻ au cation Mⁿ⁺ ou vice-versa (pat coordination du cation à l'anion).

La **figure 3** représente la méthode de greffage électrochimique par voie diazonium. Plus particulièrement, la **figure 3a** représente le greffage direct sur support solide des dérivés cylam-diazonium formés *in-situ* à partir de dérivés « cyclam C-aniline ». La **figure 3b** représente le greffage en deux étapes sur support solide des dérivés cylam.

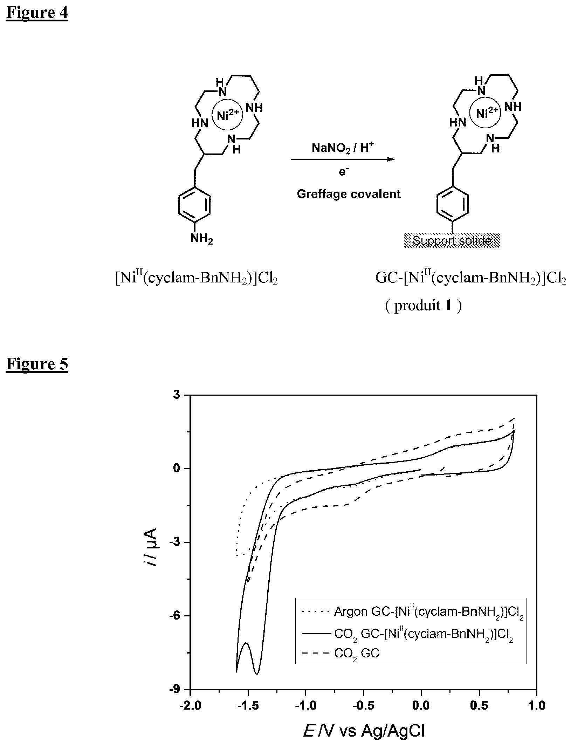

La **figure 4** est un exemple particulier de la **figure 3a** et illustre l'exemple 1. Elle représente plus particulièrement le procédé de préparation d'un produit 1 de formule générale (II) de l'invention par greffage en une seule étape par voie diazonium, du complexe [Ni^{II}(cyclam-BnNH₂)]C1₂ (BnNH₂ = benzylamine) sur une électrode de carbone vitreux (GC).

La **figure 5** (voir exemple 1) est un voltammogramme cyclique (v = 0.1 V s⁻¹) d'une électrode de carbone vitreux non fonctionnalisée sous CO₂ (tirets), et d'une électrode de carbone vitreux fonctionnalisée par le complexe [Ni^{II}(cyclam-BnNH₂)]Cl₂ (à savoir un produit de l'invention répondant à la formule générale (II) et dénommé « produit **1** ») sous atmosphère d'argon (points) et sous CO₂ (traits pleins). Les mesures sont réalisées dans CH₃CN/H₂O (4:1) + NBu₄PF₆ 0.1 M.

L'exemple suivant illustre l'invention, il ne la limite en aucune façon.

### Exemple 1

### Réduction électrocatalytique du CO₂ dans l'eau à l'aide d'un produit 1 de formule (I) de l'invention.

### 1/ Préparation d'un produit 1 de l'invention de formule (II) par voie diazonium

Le produit **1** objet de l'invention qui est préparé présente la structure générale (II) dans laquelle :
- R₁, R₂, R₃ et R₄ représentent chacun H ;
- le « support solide » est une électrode de carbone vitreux (GC) ;
- Mⁿ⁺ est Ni²⁺ ;
- la liaison « 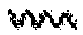 » est le radical divalent -CH₂-.

### Préparation du complexe [Ni^{II}(cyclam-BnNH₂)]Cl₂

La synthèse a été réalisée selon la procédure suivante : 100 mg de ligand Cyclam-BnNH₂ ¹⁰⁻¹² sont solubilisés dans 10 mL d'eau, et 6 équivalents de NaHCO₃ (193,9 mg) sont ajoutés à température ambiante. Après 30 minutes sous agitation, un équivalent de NiCl₂ (49,9 mg) est ajouté au mélange. La solution est agitée pendant deux heures à température ambiante. Une coloration orange apparaît. Après évaporation du solvant (eau), un solide brun-orangé est obtenu. Il est dissous dans 20 mL d'éthanol pour la purification. Après filtration, évaporation de l'éthanol et de séchage sous vide pendant une nuit, une poudre de couleur violet clair du complexe [Ni(cyclam-BnNH₂)]Cl₂ est obtenue avec un rendement de 57 % (87,1 mg).

### Préparation du produit 1 de l'invention répondant à la formule (II)

Le greffage covalent en une seule étape par voie diazonium du complexe [Ni^{II}(cyclam-BnNH₂)]C1₂ (BnNH₂ = benzylamine) sur l'électrode de carbone vitreux (GC) pour conduire au produit **1** est représenté à la **figure 4****.**

Plus particulièrement, l'électrode est plongée dans une solution dégazée (Ar, 20°C) d'acide sulfurique diluée (0,5 M) comprenant 5 mM du complexe [Ni^{II}(cyclam-BnNH₂)]C1₂ et 5,25 mM NaNO₂ (pH = 2).

Le balayage en potentiel (+ 0,4 V à - 0,8 V vs Ag/AgCl) à une vitesse de 100 mV s⁻¹ sur plusieurs cycles (typiquement 5), et/ou l'application d'un potentiel de -0,8 V pendant 100 s conduit au dépôt sur électrode.

L'électrode est rincée avec de l'eau après greffage.

### Note :

Le composé **1** de formule (I) tel qu'obtenu ci-dessus aurait aussi pû être préparé par la méthode de couplage peptidique (méthode de greffage en deux étapes) (voir **figure 3b**).

Selon la nature du groupement terminal (X = COOH, CH₂COOH ou NH₂) du précurseur greffé lors de la première étape, la procédure de couplage peut varier.

Si X=NH₂, l'électrode fonctionnalisée est plongée pendant 2 h dans une solution aqueuse contenant les trois composés EDC (40 mM), NHS (10 mM) et le cyclam carboxylé (1 mM). Si X = COOH ou CH₂COOH, l'électrode est tout d'abord plongée dans une solution EDC/NHS (40 mM/10 mM) pendant 1 h, puis dans une solution 1 mM du cyclam à terminaison amine pendant 1 h. Elle est ensuite rincée (H₂O puis EtOH).

### 2/ Réduction électrocatalytique du CO₂ à l'aide du produit 1 de l'invention

L'électrode de carbone vitreux fonctionnalisée par le complexe [Ni^{II}(cyclam-BnNH₂)]C1₂ (produit **1**), mentionnée comme GC-[Ni^{II}(cyclam-BnNH₂)]Cl₂ à la figure 5, est ensuite plongée dans une solution aqueuse (CH₃CN:H₂O, 4 :1) contenant un sel d'électrolyte (NBu₄PF₆ 0.1M) pour des études de voltammétrie cyclique sur la réduction électrocatalytique du CO₂, selon un montage classique à 3 électrodes (électrode auxiliaire : fil de Pt ; électrode de référence : Ag/AgCl/NaCl). Les études avec l'électrode GC-[Ni^{II}(cyclam-BnNH₂)]Cl₂ sont réalisées sous atmosphère inerte (Argon) et sous CO₂ (solution saturée). Pour comparaison, une électrode de carbone vitreux non fonctionnalisée, mentionnée comme GC, est utilisée. Les résulats obtenus sont illustrés à la **figure 5****.** Comme clairement démontré par les courbes de voltammétrie cyclique de cette figure, l'électrode de carbone vitreux GC fonctionnalisée par le complexe Ni-cyclam (à savoir GC-[Ni^{II}(cyclam-BnNH₂)]Cl₂ représenté par les courbes en traits pleins) présente une activité électrocatalytique pour la réduction du dioxyde de carbone en milieux aqueux (augmentation du courant par comparaison avec les expériences sous argon, courbe GC-[Ni^{II}(cyclam-BnNH₂)]Cl₂ en traits pointillés). Le pic de courant détectecté à -1.5 V est en effet caractéristique d'une réaction d'électrocatalyse. Dans les mêmes conditions, l'électrode de carbone vitreux non fonctionnalisée (GC, courbe en tirets) ne présente pas ce pic sous CO₂.

### Conclusion :

Les Inventeurs ont développé une nouvelle approche pour l'immobilisation de complexe Ni-cyclam C-fonctionnalisés sur électrodes, basée sur un greffage covalent activé par voie électrochimique par « voie diazonium ».

Les analyses spectroscopiques et électrochimiques des surfaces fonctionnalisées témoignent d'un greffage sur surface du complexe étudié.

Les complexes Ni-cyclam immobilisés démontrent des propriétés remarquables pour la réduction électrocatalytique du dioxyde de carbone en milieu aqueux. Ceci est lié à la faible distance séparant l'ion métallique de l'électrode, permettant ainsi un transfert électronique rapide.

Les Inventeurs ont également développé d'autres catalyseurs C-fonctionnalisés de formule (II) sur lesquels divers substituants ont été introduits sur le noyau cyclam dans l'objectif de diminuer la surtension pour la réduction de CO₂, mais aussi permettre l'augmentation de la cinétique de la réaction catalytique.

### Bibliographie

1. Zhanaidarova, A.; Moore, C. E.; Gembicky, M.; Kubiak, C. P., Covalent attachment of [Ni(alkynyl-cyclam)](2+) catalysts to glassy carbon électrodes. Chem Commun (Camb) 2018, 54 (33), 4116-4119.
2. Jasmin, J.-P.; Ouhenia-Ouadahi, K.; Miserque, F.; Dumas, E.; Cannizzo, C.; Chaussé, A., Straightforward grafting approach for cyclam- functionalized screen-printed électrodes for sélective Cu(II) détermination. Electrochimica Acta 2016, 200, 115-122.
3 Neri, G.; Walsh, J. J.; Wilson, C.; Reynal, A.; Lim, J. Y.; Li, X.; White, A. J.; Long, N. J.; Durrant, J. R.; Cowan, A. J., A functionalised nickel cyclam catalyst for CO2 réduction: electrocatalysis, semiconductor surface immobilisation and light-driven electron transfer. Phys Chem Chem Phys 2015, 17 (3), 1562-6.
4. Takeda, H.; Cometto, C.; Ishitani, O.; Robert, M., Electrons, Photons, Protons and Earth-Abundant Metal Complexes for Molecular Catalysis of CO2 Réduction. ACS Catalysis 2016, 7 (1), 70-88.
5. Bonin, J.; Maurin, A.; Robert, M., Molecular catalysis of the electrochemical and photochemical réduction of CO2 with Fe and Co metal based complexes. Recent advances. Coordination Chemistry Reviews 2017, 334, 184-198.
6. Francke, R.; Schille, B.; Roemelt, M., Homogeneously Catalyzed Electroreduction of Carbon Dioxide-Methods, Mechanisms, and Catalysts. Chem Rev 2018.
7. Allongue, P.; Delamar, M.; Desbat, B.; Fagebaume, O.; Hitmi, R.; Pinson, J.; Savéant, J.-M., Covalent Modification of Carbon Surfaces by Aryl Radicals Generated from the Electrochemical Réduction of Diazonium Salts. Journal of the American Chemical Society 1997, 119 (1), 201-207.
8. Pinson, J.; Podvorica, F., Attachment of organic layers to conductive or semiconductive surfaces by réduction of diazonium salts. Chem Soc Rev 2005, 34 (5), 429-39.
9. Breton, T.; Belanger, D., Modification of carbon electrode with aryl groups having an aliphatic amine by electrochemical réduction of in situ generated diazonium cations. Langmuir 2008, 24 (16), 8711-8.
10. Camus, N.; Halime, Z.; Le Bris, N.; Bernard, H.; Platas-Iglesias, C.; Tripier, R., Full control of the regiospecific N-functionalization of C-functionalized cyclam bisaminal dérivatives and application to the synthesis of their TETA, TE2A, and CB-TE2A analogues. J. Org. Chem. 2014, 79 (5), 1885-99.
11. Tripier, R.; Camus, N. Methods for preparing functional tetraazacycloalkane compounds using a spécifie cyclic bisaminal compound. WO2013072491, 2012.
12. Halime, Z.; Frindel, M.; Camus, N.; Orain, P. Y.; Lacombe, M.; Bemardeau, K.; Cherel, M.; Gestin, J. F.; Faivre-Chauvet, A.; Tripier, R., New synthesis of phenyl-isothiocyanate C-functionalised cyclams. Bioconjugation and (64)Cu phenotypic PET imaging studies of multiple myeloma with the te2a dérivative. Org. Biomol. Chem. 2015, 13 (46), 11302-14.

## Revendications

1. Produit **caractérisé en ce qu'**il présente la structure de formule générale (I) suivante : 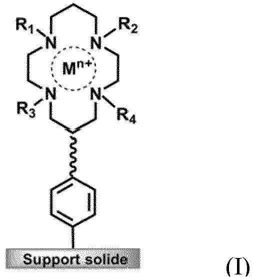 dans laquelle
• R₁, R₂, R₃ et R₄ représentent indépendamment l'un de l'autre H ; un alkyle en C₁-C₅ ; (CH₂)ₘR' avec m égal à un entier allant de 0 à 3 et R' représentant C₆H₅ (Ph), NO₂, CO₂(alkyle en C₁-C₃), O(alkyle en C₁-C₃), un halogène choisi parmi Cl, F, Br ou I;
• « Support solide » représente un matériau conducteur, notamment une électrode ou des nanoparticules ;
• la liaison « 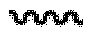 » entre le carbone du macrocycle 1,4,8,11-tétraazacyclotétradécane (cyclam) et le carbone du groupement C₆H₄ représente le radical divalent -(CH₂)ₘ- avec m égal à un entier allant de 0 à 3 ; -CO-NH- ; -NH-CO- ;
• la liaison « » entre le carbone du groupement C₆H₄ et le support solide est une liaison covalente ;
• Mⁿ⁺, s'il est présent, représente un cation métallique, avec n qui est un entier allant de 1 à 4 et M est choisi dans le groupe comprenant Ni, Cu, Hg, Zn, Co, Cd et Pb.

2. Produit selon la revendication 1, **caractérisé en ce que** R₁, R₂, R₃ et R₄ représentent indépendamment l'un de l'autre H ou un alkyle en C₁-C₅, ledit alkyle étant de préférence un méthyle.

3. Produit selon la revendication 1 ou la revendication 2, **caractérisé en ce que** le « support solide » est une électrode choisie dans le groupe comprenant une électrode de carbone, d'or, de platine, d'oxyde d'étain dopé au fluor (« FTO » acronyme anglais de « Fluorine doped Tin oxide »), d'oxyde d'étain dopé à l'indium (« ITO » acronyme anglais de « Indium doped Tin oxide »), et est de préférence une électrode de carbone ou d'or.

4. Produit selon la revendication 3, **caractérisé en ce que** la liaison covalente « » entre le carbone du groupement C₆H₄ et le support solide est une liaison de type « carbone-carbone » ou « carbone-hétéroatome », ledit hétéroatome étant un métal, de préférence l'or.

5. Produit selon l'une quelconque des revendications 1 à 4, **caractérisé en ce que** Mⁿ⁺, s'il est présent, représente un cation métallique choisi dans le groupe comprenant Ni²⁺, Cu²⁺, Hg²⁺, Zn²⁺, Co²⁺, Cd²⁺ et Pb²⁺, et est de préférence Ni²⁺ ou Cu²⁺.

6. Produit selon l'une quelconque des revendications 1 à 5, **caractérisé en ce que** liaison « 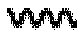 » entre le carbone du cyclam et le carbone du groupement C₆H₄ est le radical divalent -CH₂-.

7. Produit selon l'une quelconque des revendications 1 à 6, **caractérisé en ce que** dans la structure de formule (I) :
• R₁, R₂, R₃ et R₄ représentent indépendamment l'un de l'autre H ou un alkyle en C₁-C₅, ledit alkyle étant de préférence un méthyle ;
• le « support solide » est une électrode de carbone ou d'or ;
• Mⁿ⁺, s'il est présent, représente Ni²⁺ ou Cu²⁺ ;
• la liaison « 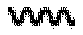 » est le radical divalent -CH₂-.

8. Produit selon l'une quelconque des revendications 1 à 7, **caractérisé en ce que** dans la structure de formule (I), Mⁿ⁺ est présent, ladite structure de formule générale (I) étant alors plus particulièrement représentée par la structure de formule (II) générale suivante : 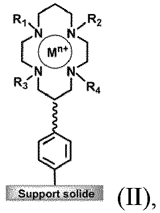 dans laquelle R₁, R₂, R₃, R₄, le « support solide », Mⁿ⁺ et la liaison « » sont tels que définis à l'une quelconque des revendications 1 à 7.

9. Produit selon l'une quelconque des revendications 1 à 7, **caractérisé en ce que** dans la structure de formule (I) Mⁿ⁺ est absent, ladite structure de formule générale (I) étant alors plus particulièrement représentée par la structure de formule (III) générale suivante : 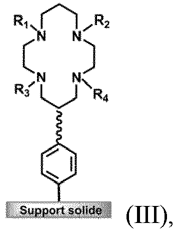 dans laquelle R₁, R₂, R₃, R₄, le « support solide » et la liaison « » sont tels que définis à l'une quelconque des revendications 1 à 4 et 6 à 7.

10. Utilisation d'un produit selon l'une quelconque des revendications 1 à 8, pour la catalyse, et plus particulièrement l'électrocatalyse.

11. Utilisation selon la revendication 10, pour la réduction électrocatalytique en milieu aqueux du dioxyde de carbone (CO₂) en monoxyde de carbone (CO).

12. Utilisation selon la revendication 10, pour l'oxydation électrocatalytique de l'eau (H₂O) en dioxygène (O₂).

13. Utilisation d'un produit selon l'une quelconque des revendications 1 à 8, pour la détection et/ou capture d'anions choisis dans le groupe comprenant des halogénures, à savoir Cl⁻, Br⁻ et F⁻ ; des ions nitrate (NO₃⁻) ; des ions hydrogénophosphate (HPO₄²⁻) et des ions sulfate (SO₄²⁻).

14. Utilisation d'un produit selon l'une quelconque des revendications 1 à 7 ou 9, pour la détection et/ou capture de cations Mⁿ⁺ dans lesquels n est un entier allant de 1 à 4 et M est choisi dans le groupe comprenant Ni, Cu, Hg, Zn, Co, Cd et Pb, et de préférence Ni²⁺ ou Cu²⁺.

## Patentansprüche

1. Erzeugnis, **dadurch gekennzeichnet, dass** es die folgende allgemeine Strukturformel (I) aufweist: 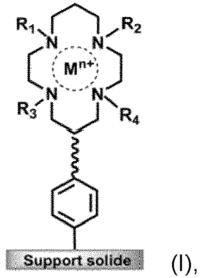 wobei:
- R₁, R₂, R₃ und R₄ unabhängig voneinander H; ein Alkyl mit C₁-C₅; (CH₂)ₘR' mit m gleich einer von 0 bis 3 laufenden Ganzzahl und wobei R' C₆H₅ (Ph) repräsentiert, NO₂, COz(Alkyl mit C₁-C₃), O (Alkyl mit C₁-C₃), ein aus Cl, F, Br oder I ausgewähltes Halogen repräsentieren;
- "Support solide", fester Träger, ein leitfähiges Material repräsentiert, insbesondere eine Elektrode oder Nanopartikel;
- die Bindung "~~~" zwischen dem Kohlenstoff des makrozyklischen 1, 4, 8, 11-Tetraazacyclotetradecan (Cyclam) und dem Kohlenstoff der C₆H₄-Gruppe das divalente Radikal -(CH₂)ₘ- mit m gleich einer von 0 bis 3 laufenden Ganzzahl; -CO-NH-; -NH-CO- repräsentiert;
- die Bindung "---" zwischen dem Kohlenstoff der C₆H₄-Gruppe und dem festen Träger eine kovalente Bindung ist;
- Mⁿ⁺, wenn es vorliegt, ein metallisches Kation repräsentiert, wobei n eine von 1 bis 4 laufende Ganzzahl ist und M aus der Gruppe ausgewählt ist, welche Ni, Cu, Hg, Zn, Co, Cd und Pb umfasst.

2. Erzeugnis nach Anspruch 1, **dadurch gekennzeichnet, dass** R₁, R₂, R₃ und R₄ unabhängig voneinander H oder ein Alkyl mit C₁-C₅ repräsentieren, wobei das Alkyl vorzugsweise ein Methyl ist.

3. Erzeugnis nach Anspruch 1 oder Anspruch 2, **dadurch gekennzeichnet, dass** der "feste Träger" eine Elektrode ist, ausgewählt aus der Gruppe, umfassend eine Elektrode aus Kohlenstoff, aus Gold, aus Platin, aus Fluor-dotiertem Zinnoxid ("FTO" englische Abkürzung für "Fluorine doped Tin oxide"), aus Indium-dotiertem Zinnoxid ("ITO" englische Abkürzung für "Indium doped Tin oxide"), und vorzugsweise eine Elektrode aus Kohlenstoff oder Gold ist.

4. Erzeugnis nach Anspruch 3, **dadurch gekennzeichnet, dass** die kovalente Bindung "---" zwischen der C₆H₄-Gruppe und dem festen Träger eine Bindung vom Typ "Kohlenstoff-Kohlenstoff" oder "Kohlenstoff-Heteroatom" ist, wobei das Heteroatom ein Metall ist, vorzugsweise Gold.

5. Erzeugnis nach einem der Ansprüche 1 bis 4, **dadurch gekennzeichnet, dass** Mⁿ⁺, wenn es vorliegt, ein metallisches Kation repräsentiert, ausgewählt aus der Gruppe, umfassend Ni²⁺, Cu²⁺, Hg²⁺, Zn²⁺, Co²⁺, Cd²⁺ und Pb²⁺, und vorzugsweise Ni²⁺ oder Cu²⁺ ist.

6. Erzeugnis nach einem der Ansprüche 1 bis 5, **dadurch gekennzeichnet, dass** die Bindung "~~~" zwischen dem Kohlenstoff des Cyclam und dem Kohlenstoff der C₆H₄-Gruppe das divalente Radikal -CH₂- ist.

7. Erzeugnis nach einem der Ansprüche 1 bis 6, **dadurch gekennzeichnet, dass** in der Strukturformel (I):
- R₁, R₂, R₃ und R₄ unabhängig voneinander H oder ein Alkyl mit C₁-C₅ repräsentieren, wobei das Alkyl vorzugsweise ein Methyl ist;
- der "feste Träger" eine Elektrode aus Kohlenstoff oder aus Gold ist;
- Mⁿ⁺, falls es vorliegt, Ni²⁺ oder Cu²⁺ repräsentiert;
- die Bindung "~~~" das divalente Radikal -CH₂- ist.

8. Erzeugnis nach einem der Ansprüche 1 bis 7, **dadurch gekennzeichnet, dass** in der Strukturformel (I) Mⁿ⁺ vorliegt, wobei die allgemeine Strukturformel (I) demnach insbesondere durch die folgende allgemeine Strukturformel (II) repräsentiert wird: 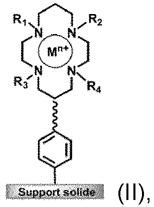 wobei R₁, R₂, R₃, R₄, der "feste Träger", Mⁿ⁺ und die Bindung "~~~" wie in einem der Ansprüche 1 bis 7 definiert sind.

9. Erzeugnis nach einem der Ansprüche 1 bis 7, **dadurch gekennzeichnet, dass** in der Strukturformel (I) Mⁿ⁺ abwesend ist, wobei die allgemeine Strukturformel (I) demnach insbesondere durch die folgende allgemeine Strukturformel (III) repräsentiert wird: 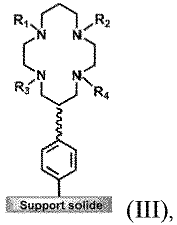 wobei R₁, R₂, R₃, R₄, der "feste Träger" und die Bindung "~~~" wie in einem der Ansprüche 1 bis 4 und 6 bis 7 definiert sind.

10. Verwendung eines Erzeugnisses nach einem der Ansprüche 1 bis 8 für die Katalyse, insbesondere die Elektrokatalyse.

11. Verwendung nach Anspruch 10 für die elektrokatalytische Reduktion in wässriger Umgebung von Kohlendioxid (CO₂) zu Kohlenmonoxid (CO).

12. Verwendung nach Anspruch 10 für die elektrokatalytische Oxidation von Wasser (H₂O) zu Di-Sauerstoff (O₂)

13. Verwendung eines Erzeugnisses nach einem der Ansprüche 1 bis 8 für die Detektion und/oder den Einfang von Anionen, ausgewählt aus der Gruppe, umfassend Halogenide, das heißt Cl⁻, Br und F-; Nitrat-Ionen (NO₃⁻); Hydrogenphosphat-Ionen (HPO₄²⁻) und Sulfat-Ionen (SO₄²⁻).

14. Verwendung eines Erzeugnisses nach einem der Ansprüche 1 bis 7 oder 9 für die Detektion und/oder den Einfang von Kationen Mⁿ⁺, wobei n eine von 1 bis 4 laufende Ganzzahl ist und M ausgewählt ist aus der Gruppe, umfassend Ni, Cu, Hg, Zn, Co, Cd und Pb, und bevorzugt Ni²⁺ oder Cu²⁺.

## Claims

1. Product, **characterized in that** it has the structure of general formula (I) below: 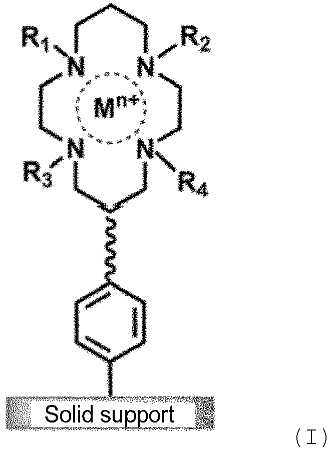 in which
• R₁, R₂, R₃ and R₄ represent, independently of one another, H; a C₁-C₅ alkyl; (CH₂)ₘR' with m equal to an integer ranging from 0 to 3 and R' representing C₆H₅ (Ph), NO₂, CO₂ (C₁-C₃ alkyl), O(C₁-C₃ alkyl), a halogen selected from Cl, F, Br or I;
• "Solid support" represents a conductive material, in particular an electrode or nanoparticles;
• the bond " 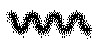 " between the carbon of the 1,4,8,11-tetraazacyclotetradecane macrocycle (cyclam) and the carbon of the C₆H₄ group represents the divalent radical -(CH₂)ₘ- with m equal to an integer ranging from 0 to 3; -CO-NH-; -NH-CO-;
• the bond " 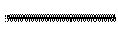 " between the carbon of the C₆H₄ group and the solid support is a covalent bond;
• Mⁿ⁺, if it is present, represents a metal cation, with n which is an integer ranging from 1 to 4 and M is selected from the group comprising Ni, Cu, Hg, Zn, Co, Cd and Pb.

2. Product according to Claim 1, **characterized in that** R₁, R₂, R₃ and R₄ represent, independently of one another, H or a C₁-C₅ alkyl, said alkyl preferably being a methyl.

3. Product according to Claim 1 or Claim 2, **characterized in that** the "solid support" is an electrode selected from the group comprising a carbon, gold, platinum, fluorine-doped tin oxide ("FTO") or indium-doped tin oxide ("ITO") electrode, and is preferably a carbon or gold electrode.

4. Product according to Claim 3, **characterized in that** the covalent bond " " between the carbon of the C₆H₄ group and the solid support is a bond of the "carbon-carbon" or "carbon-heteroatom" type, said heteroatom being a metal, preferably gold.

5. Product according to any one of Claims 1 to 4, **characterized in that** Mⁿ⁺, if it is present, represents a metal cation selected from the group comprising Ni²⁺, Cu²⁺, Hg²⁺, Zn²⁺, Co²⁺, Cd²⁺ and Pb²⁺, and is preferably Ni²⁺ or Cu²⁺.

6. Product according to any one of Claims 1 to 5, **characterized in that** the bond " 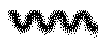 " between the carbon of the cyclam and the carbon of the C₆H₄ group is the divalent radical -CH₂-.

7. Product according to any one of Claims 1 to 6, **characterized in that** in the structure of formula (I):
• R₁, R₂, R₃ and R₄ represent, independently of one another, H or a C₁-C₅ alkyl, said alkyl preferably being a methyl;
• the "solid support" is a carbon or gold electrode;
• Mⁿ⁺, if it is present, represents Ni²⁺ or Cu²⁺;
• the bond " 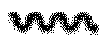 " is the divalent radical -CH₂-.

8. Product according to any one of Claims 1 to 7, **characterized in that** in the structure of formula (I), Mⁿ⁺ is present, said structure of general formula (I) then being more particularly represented by the structure of general formula (II) below: 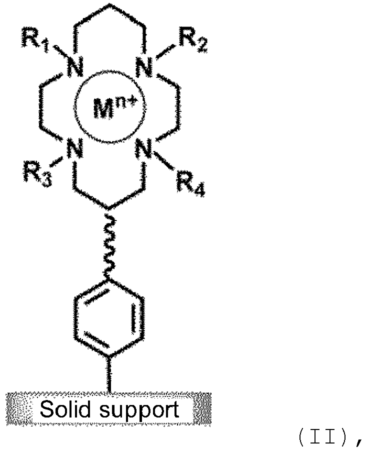 in which R₁, R₂, R₃, R₄, the "solid support", Mⁿ⁺ and the bond " 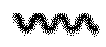 " are as defined in any one of Claims 1 to 7.

9. Product according to any one of Claims 1 to 7, **characterized in that** in the structure of formula (I) Mⁿ⁺ is absent, said structure of general formula (I) then being more particularly represented by the structure of general formula (III) below: 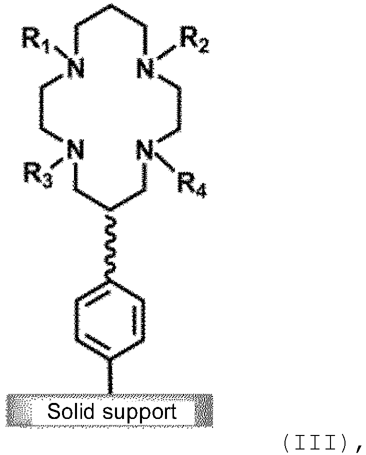 in which R₁, R₂, R₃, R₄, the "solid support" and the bond " 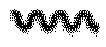 " are as defined in any one of Claims 1 to 4 and 6 to 7.

10. Use of a product according to any one of Claims 1 to 8, for catalysis, and more particularly electrocatalysis.

11. Use according to Claim 10, for the electrocatalytic reduction in aqueous medium of carbon dioxide (CO₂) to carbon monoxide (CO).

12. Use according to Claim 10, for the electrocatalytic oxidation of water (H₂O) to dioxygen (O₂) .

13. Use of a product according to any one of Claims 1 to 8, for the detection and/or capture of anions selected from the group comprising halides, namely Cl⁻, Br⁻ and F⁻ ; nitrate ions (NO₃⁻) ; hydrogen phosphate ions (HPO₄²⁻) and sulfate ions (SO₄²⁻) .

14. Use of a product according to any one of Claims 1 to 7 or 9, for the detection and/or capture of Mⁿ⁺ cations in which n is an integer ranging from 1 to 4 and M is selected from the group comprising Ni, Cu, Hg, Zn, Co, Cd and Pb, and preferably Ni²⁺ or Cu²⁺.
